# EUROPEAN PATENT APPLICATION

(11) **EP 2 353 607 A1**
(43) Date of publication of application: **10.08.2011**
(21) Application number: 10152698.6
(22) Date of filing: 04.02.2010
(51) Int. Cl.: A61K 38/48, A61K 38/17, A61P 17/06, A61P 29/00

(54) **Use of disintegrin domain of an adamalysin for the treatment of psoriasis**

(71) Applicant: BioAlliance Pharma, 75015 Paris (FR)
(72) Inventor: The designation of the inventor has not yet been filed
(74) Representative: Colombet, Alain André

(57) **Abstract**

The present invention is related to the treatment of psoriasis or other chronic inflammatory diseases using the disintegrin domain of Adamalysin. More particularly, the invention discloses psoriasis treatment by electrically mediated gene transfer of disintegrin domain of Adamalysin.

## Description

### TECHNICAL FIELD

The present invention is related to the treatment of psoriasis or other chronic inflammatory diseases. More particularly, the invention discloses psoriasis treatment by electrically mediated gene transfer of disintegrin domain of Adamalysin.

### BACKGROUND

Psoriasis is a chronic, non-contagious disease that affects mainly the skin. It commonly causes red, scaly patches to appear on the skin, although some patients have no dermatological symptoms. The scaly patches caused by psoriasis, called psoriatic plaques, are areas of inflammation and excessive skin production. Skin rapidly accumulates at these sites and takes on a silvery-white appearance. Plaques frequently occur on the skin of the elbows and knees, but can affect any area including the scalp, palms of hands and soles of feet, and genitals. The disorder is a chronic recurring condition that varies in severity from minor localized patches to complete body coverage.

The symptoms of psoriasis can manifest in a variety of forms:
- Plaque psoriasis (psoriasis vulgaris) is the most common form of psoriasis. It affects 80 to 90% of people with psoriasis. Plaque psoriasis typically appears as raised areas of inflamed skin covered with silvery white scaly skin. These areas are called plaques.
- Flexural psoriasis (inverse psoriasis) appears as smooth inflamed patches of skin. It occurs in skin folds, particularly around the genitals (between the thigh and groin), the armpits, under an overweight stomach (pannus), and under the breasts (inframammary fold). It is aggravated by friction and sweat, and is vulnerable to fungal infections.
- Guttate psoriasis is characterized by numerous small round spots (differential diagnosis—pityriasis rosea—oval shape lesion). These numerous spots of psoriasis appear over large areas of the body, such as the trunk, limbs, and scalp. Guttate psoriasis is associated with streptococcal throat infection.
- Pustular psoriasis appears as raised bumps that are filled with non-infectious pus (pustules). The skin under and surrounding the pustules is red and tender. Pustular psoriasis can be localised, commonly to the hands and feet (palmoplantar pustulosis), or generalised with widespread patches occurring randomly on any part of the body.
- Nail psoriasis produces a variety of changes in the appearance of finger and toe nails. These changes include discolouring under the nail plate, pitting of the nails, lines going across the nails, thickening of the skin under the nail, and the loosening (onycholysis) and crumbling of the nail.
- Erythrodermic psoriasis involves the widespread inflammation and exfoliation of the skin over most of the body surface. It may be accompanied by severe itching, swelling and pain. It is often the result of an exacerbation of unstable plaque psoriasis, particularly following the abrupt withdrawal of systemic treatment. This form of psoriasis can be fatal, as the extreme inflammation and exfoliation disrupt the body's ability to regulate temperature and for the skin to perform barrier functions.
- Psoriatic arthritis involves joint and connective tissue inflammation. Psoriatic arthritis can affect any joint but is most common in the joints of the fingers and toes. This can result in a sausage-shaped swelling of the fingers and toes known as dactylitis. Psoriatic arthritis can also affect the hips, knees and spine (spondylitis). About 10-15% of people who have psoriasis also have psoriatic arthritis.

The cause of psoriasis is not well understood. There are two main hypotheses about the process that occurs in the development of the disease. The first considers psoriasis as primarily a disorder of excessive growth and reproduction of skin cells. The problem is simply seen as a fault of the epidermis and its keratinocytes. The second hypothesis sees the disease as being an immune-mediated disorder in which the excessive reproduction of skin cells is secondary to factors produced by the immune system. T cells (which normally help protect the body against infection) become active, migrate to the dermis and trigger the release of cytokines (tumor necrosis factor-alpha TNFα, in particular) which cause inflammation and the rapid production of skin cells. It is not known what initiates the activation of the T cells.

Factors that may aggravate psoriasis include stress, withdrawal of systemic corticosteroid, excessive alcohol consumption, and smoking. There are many treatments available, but because of its chronic recurrent nature psoriasis is a challenge to treat.

The current drugs for the treatment of psoriasis follow two strategies: anti-T cell strategies and anti-cytokine strategies.

Two drugs that target T cells are efalizumab and alefacept. Efalizumab is a monoclonal antibody which blocks the molecules that dendritic cells use to communicate with T cells. It also blocks the adhesion molecules on the endothelial cells that line blood vessels, which attract T cells. However, it suppressed the immune system's ability to control normally harmless viruses, which led to fatal brain infections. Alefacept also blocks the molecules that dendritic cells use to communicate with T cells, and even causes natural killer cells to kill T cells, as a way of controlling inflammation.

Several monoclonal antibodies (MABs) target cytokines, the molecules that cells use to send inflammatory signals to each other. One of the main inflammatory signals in the body is tumor necrosis factor α (TNF-α), and three MABs -- infliximab, adalimumab and etanercept bind to TNF-α. Two more inflammatory signals are interleukin-23 and interleukin-12. A protein chain, p40, is the same on both of those interleukins, and the monoclonal antibody ustekinumab binds to that common protein to interfere with both of them.

There can be substantial variation between individuals in the effectiveness of specific psoriasis treatments. Because of this, dermatologists often use a trial-and-error approach to finding the most appropriate treatment for their patient. The decision to employ a particular treatment is based on the type of psoriasis, its location, extent and severity. The patient's age, sex, quality of life, comorbidities, and attitude toward risks associated with the treatment are also taken into consideration.

In 2008, the FDA (Food and Drug Administration) approved three new treatment for psoriasis: 1) Taclonex Scalp, a new topical ointment for treating scalp psoriasis; 2) the Xtrac Velocity excimer laser system, which emits a high-intensity beam of ultraviolet light, can treat moderate to severe psoriasis; and 3) the biologic drug adalimumab was also approved to treat moderate to severe psoriasis. Adalimumab had already been approved to treat psoriatic arthritis.

Medications with the least potential for adverse reactions are preferentially employed. If the treatment goal is not achieved then therapies with greater potential toxicity may be used. Medications with significant toxicity are reserved for severe unresponsive psoriasis. As a first step, topical treatments are applied to the skin. If topical treatment fails to achieve the desired goal then the next step would be to expose the skin to ultraviolet (UV) radiation (phototherapy). The third step involves the use of medications which are taken internally by pill or injection (systemic treatment).

Over time, psoriasis becomes resistant to a specific therapy. Treatments must be periodically changed to prevent resistance developing (tachyphylaxis) and to reduce the chance of adverse reactions occurring. New treatments of Psoriasis are thus still needed.

Dysregulated angiogenesis is emerging as a potential new target in inflammatory disorders, among which psoriasis (Detmar, M. et al. "Overexpression of vascular permeability factor/vascular endothelial growth factor and its receptors in psoriasis." J. Exp. Med 1994 ; Simonetti, O and al. "VEGF is likely a key factor in the link between inflammation and angiogenesis in psoriasis: results of an immunohistochemical study" Int. J. Immunopathol. Pharmacol, 2001). It is thought that the pro-angiogenic microenvironment within psoriatic skin is induced by a T-helper cell-initiated inflammatory response, which results in induction and activation of various pro-angiogenic factors such as VEGF, HIF, TNFα and CXCL-8 (Costa, C and al. "Angiogenesis and chronic inflammation: cause or consequence?" Angiogenesis 2007 Simonetti, O. and al.. VEGF is likely a key factor in the link between inflammation and angiogenesis in psoriasis: results of an immunohistochemical study. Int. J. Immunopathol. Pharmacol 2006). However, *in vivo* proof of such concept is still lacking in the state of the art (Halin, C. and al.. "Inflammation, angiogenesis, and lymphangiogenesis." Methods Enzymol. 2008 ; Saraceno, R. , and al.. Recent patents and new strategies in the treatment of psoriasis. Recent Pat. Antiinfect. Drug Discov 2006).

The family of adamalysin proteins, also referred to as A Disintegrin And Metalloproteinase Proteins (ADAMs), contains a disintegrin domain located on the metalloproteinase domain. The disintegrin region contains an integrin-binding sequence that interacts with integrins and may mediate cell-cell interactions (Wolfsberg, J Cell Biol 1995;131:275-8). Metargidin (metalloprotease-RGDdisintegrin protein), also called human ADAM-15, is a transmembrane adamalysin expressed by smooth muscle cells, mesangial cells, and at a much higher level, activated angiogenic endothelial cells (Kratzschmar et al., J Biol Chem 1996;271:4593-6; Ham et al., Exp Cell Res 2002;279:239-47; Herren et al., FASEB J 1997;11:173-80; Martin et al., J Biol Chem 2002;277:33683-9).

AMEP (for Antiangiogenic Metargidin Peptide) is the recombinant disintegrin domain (RDD) of metargidin. The AMEP peptide is the only adamalysin known to bind integrins α₅β₁ and αᵥβ₃, some receptors known to be crucial for angiogenesis (Zhang et al., J Biol Chem 1998;273:7345-50; Nath et al., J Cell Sci 1999;112:579-87), via the RGDC integrin-binding sequence, suggesting that the functions of these integrins and metargidin may be mutually dependent. The hypothesis that AMEP, expressed as soluble recombinant protein, might prevent this interaction led to explore the action of RDD on angiogenesis and tumor growth.

The adamalysins are further described in international application WO03009866, incorporated herein by reference.

Electrically mediated gene transfer, also termed DNA electrotransfer or electrogenetherapy, has gained a real interest as it is one of the most effective methods of in vivo non-viral gene transfer (Andre and Mir, 2004). The method has been shown to be effective to electrotransfer plasmid DNA to various tissues: muscles (Mir and al., 1999), liver (Suzuki et al., 1998), skin (Zhang et al., 1996), tumors (Heller and Coppola, 2002), mouse testis (Muramatsu et al., 1998), etc (Andre and Mir, 2004).

The mechanisms by which electric pulses mediate DNA transfer into target cells are not well understood. Nevertheless, there is a common agreement that for an improved DNA transfer into tissues, cells in that tissue must be permeabilized. Such a permeabilization can be achieved using simple runs of short square wave electric pulses (in the range of 100 µs) (Miklavcic et al., 2000). This kind of pulses has been widely used for the local delivery of nonpermeant anticancer drugs (like bleomycin or cisplatin) in a treatment termed 'antitumor electrochemotherapy (Mir et al., 1991 ; Rodriguez et al., 2002). Indeed, the delivery to tumors of e.g. 8 pulses of 1300 V/cm and 100 µs either *in vitro* or *in vivo* is sufficient to induce transient rearrangements of the cell membrane that allow non-permeant anticancer molecules like bleomycin to enter the cell by diffusion and to fully exert their cytotoxic activity (Mir et al., 1991; Gehl et al., 1998).

These short permeabilizing electric pulses have also been shown to increase the transfer of plasmid DNA into several tissues (Heller et al., 2000). However, another type of square-wave electric pulses was applied to muscles (Mir et al., 1999), tumors (Rols et al., 1998), liver (Suzuki et al., 1998) and some other tissues (Andre and Mir, 2004), and was found to be more effective for DNA electrotransfer (Mir et al., 1999; Heller et al., 2000). These pulses usually are of lower voltage but much longer duration (in the range of tens of milliseconds) (Mir et al., 1999; Bettan et al., 2000; Matsumoto et al., 2001). It is assumed that this type of pulses mediate DNA transfer into the cells by inducing two distinct effects that include cell permeabilization (like the short pulses) and DNA electrophoretic migration during the delivery of the electric field (Mir et al., 1999; Golzio et al., 2002).

The double role of the electric pulses on *in vivo* DNA electrotransfer was demonstrated by using combinations of electric pulses consisting of high voltage, short pulses (or HV; e.g. 800 V/cm and 100 µs) followed by low voltage, long pulses (or LV; e.g. 80 V/cm and 100 ms) (Satkauskas et al., 2002). In this last study it has been shown that these HV and LV pulses can be separated by various lags between the HV and the LV(s) without significant loss in transfection efficiency. These lags ranged up to 300 s for 1 HV and 1 LV, and up to 3000 s for 1 HV and 4LV combinations (Satkauskas etal., 2002).

However, such electrotransfer method can induce damage to the overlying skin and / or other adjacent tissue (Villemejane, J., and Al. "Physical methods of nucleic acid transfer: general concepts and applications" Br. J. Pharmacol. 2009 ; Hojman, P. and al.. Gene expression profiles in skeletal muscle after gene electrotransfer. BMC Mol. Biol).

### SUMMARY OF THE INVENTION

The present invention is related to a new and inventive treatment for Psoriasis and other inflammatory disorders, solving part or all of the drawbacks in the art.

It has surprisingly been shown for the first time that halting angiogenesis by electrotransfer of nucleic acid coding for disintegrin domain of adamalysin protein can efficiently treat psoriasis and, other inflammatory disorders.

A first object of the invention is thus an active agent for its use in a method to treat and/or prevent psoriasis or other inflammatory diseases. Said active agent is selected among a protein substance comprising or constituted by all or part of the disintegrin domain of an adamalysin or a derivative thereof, or a nucleic acid molecule comprising or constituted by a polynucleotide sequence coding all or part of the disintegrin domain of an adamalysin or a derivative thereof.

A second object of the invention is a method for preventing and/or treating psoriasis or other inflammatory diseases, wherein one administer to a patient in need thereof, an active agent is selected among a polypeptide sequence comprising or constituted by all or part of the disintegrin domain of an adamalysin or a derivative thereof, or a nucleic acid molecule comprising or constituting by a polynucleotide sequence coding all or part of the disintegrin domain of an adamalysin or a derivative thereof.

In a preferred embodiment, the active agent is administered through electrotransfer. In an embodiment, the protein substance or the nucleic acid molecule in contact with cells *in vivo,* e.g. a tissue, is submitted to an electrotransfer protocol. In an embodiment, the nucleic acid molecule is contained in, or is made of, an expression vector, such as a plasmid.

In an embodiment, the protein substance in contact with cells *in vivo,* e.g. a tissue is present in a sufficient amount to get the therapeutic effect.

In another embodiment, the nucleic acid molecule in contact with cells *in vivo,* e.g. a tissue, is in a sufficient amount in order to perform a sufficient amount of expression to get the therapeutic effect.

In an embodiment, the protein substance or the nucleic acid molecule is administered to the patient through topical administration or parenteral administration, such as injection in the region of a joint or connective tissue.

In a preferred object of the invention, the adamalysin is metargidin.

In a more preferred aspect, the disintegrin domain of an adamalysin is a nucleic acid molecule comprising a polynucleotide sequence coding all or part of the disintegrin domain of metargidin, the nucleic acid sequence of which is represented in SEQ ID NO: 1 or a derivative thereof. The coding sequence of this domain is constituted by 276 nucleotides (Met-420 to Glu-511).

In another more preferred aspect, the disintegrin domain of an adamalysin a protein substance comprising or constituted in part or entirely by the disintegrin domain of metargidin, the amino acid sequence of which is represented in SEQ ID NO: 2 or a derivative thereof.

A further object of the invention is an electrotransfer method of disintegrin domain of an adamalysin as described above, comprising:
- at least one pulse of a High Voltage (HV) field strength of between 200 and 2000 volts/cm for a duration of 10 to 1000 µs and;
- at least one second pulse of Low Voltage (LV) field strength of between 50 and 200 volts/cm and of duration of between 10 and 2000 ms.

Preferred strengths and durations are further described in international application W02007/026236 incorporated herein by reference.

In an embodiment, the at least one second pulse is 1-15 pulses, preferably 2-10 pulses, more preferably 6-10 pulses, typically 8 pulses.

In an embodiment, the Low Voltage (LV) field strength is of 50-200, preferably 80-120, typically about 100 volts/cm.

In an embodiment, the duration of the Low Voltage pulse(s) is between 10 and 2000 ms, preferably 10 and 500 ms, more preferably 10 and 50 ms, typically about 50 ms.

A particularly preferred electrotransfer method uses:
- one pulse of High Voltage (HV) field strength of about 700 volts/cm for a duration of about 100 µs and
- 8 pulses of Low Voltage (LV) field strength of about 100 volts/cm and of duration of about 50 ms.

This combination of pulses facilitates gentle cell permeabilization followed by electrophoresis of the plasmids into the cells, while largely avoiding damage to the overlying skin and/or other adjacent tissues.

The use and methods according to the invention may be used to prevent and/or treat any form or symptom of psoriasis, especially those selected from the group consisting of Plaque psoriasis, Flexural psoriasis, Guttate psoriasis, Pustular psoriasis, Nail psoriasis, Erythrodermic psoriasis, Psoriatic arthritis and any combinations thereof. A preferred object of the invention is the prevention and/or treatment of Psoriatic arthritis.

The disintegrin domain of adamalysin protein is preferably used in the form of a cDNA, inserted in plasmids.

In an embodiment, the plasmid contains the human cytomegalovirus (CMV) promoter or any fragment or derivative thereof driving a constitutive expression of the transgene. In a preferred embodiment, the plasmid contains further an antibiotic resistance gene such as the kanamycine resistance gene.

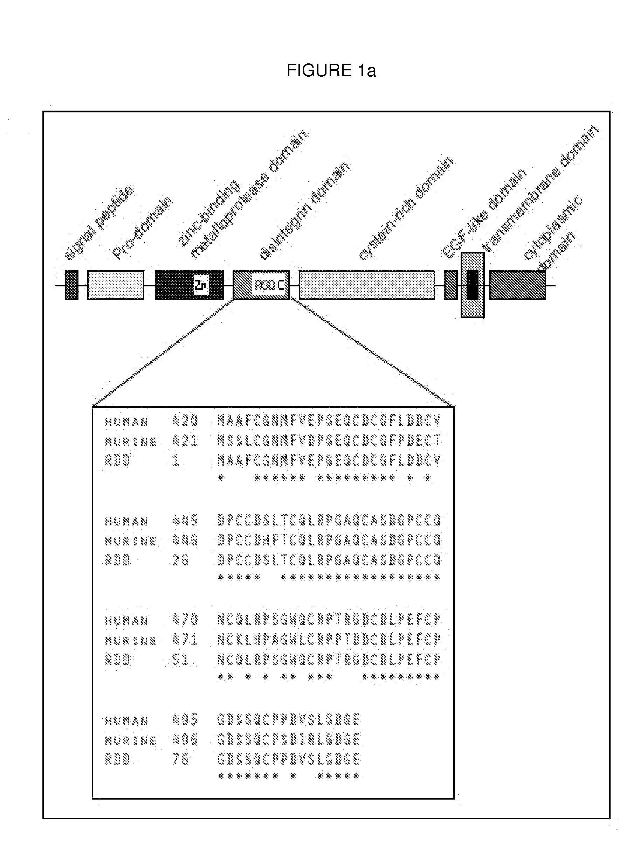

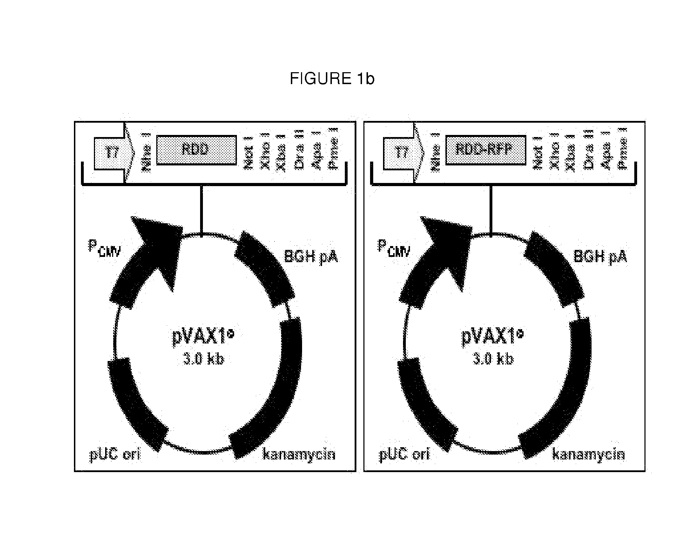

In a typical embodiment, the plasmid is a pVAX plasmid (Invitrogen, V260-20),containing the human cytomegalovirus (CMV) promoter driving a constitutive expression of the transgene, and the kanamycine resistance gene (Figure 1 a and 1 b).

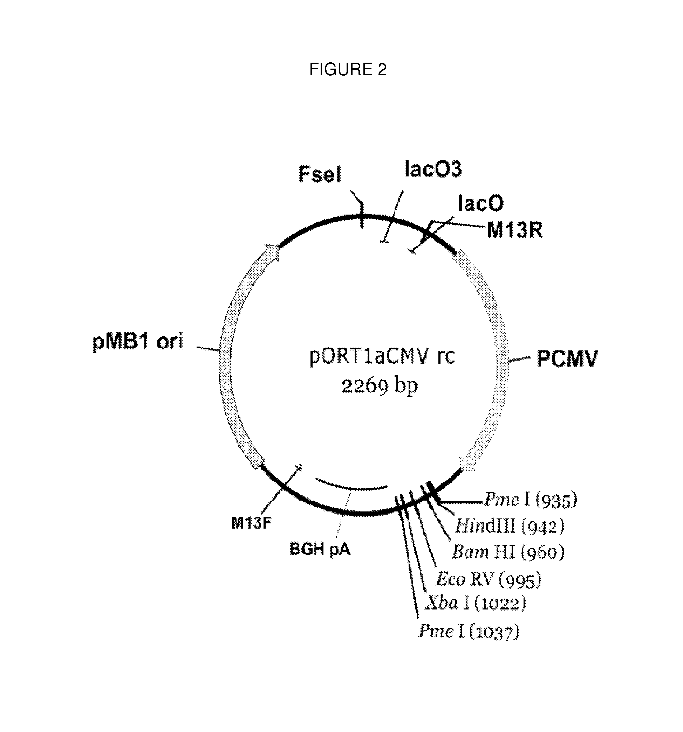

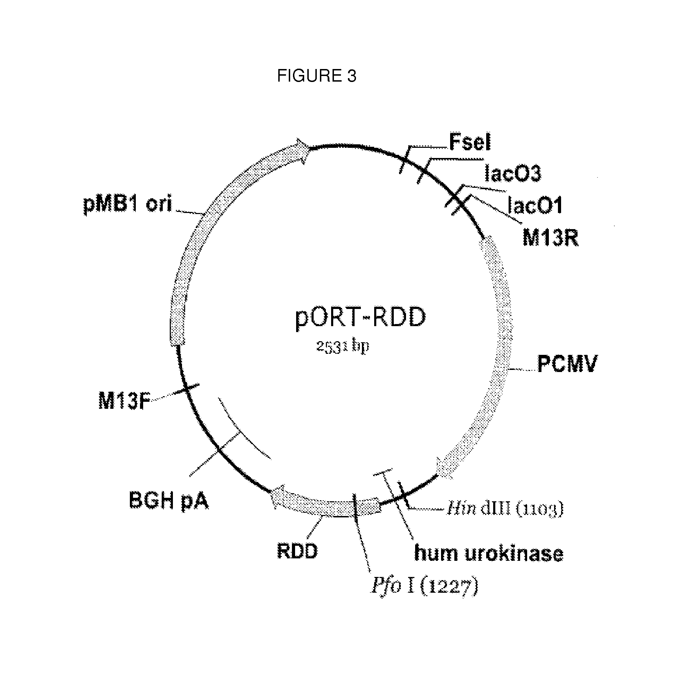

In another embodiment, the plasmid does not contain an antibiotic resistance gene. An advantageous plasmid is a plasmid devoid of any antibiotic resistance gene, dedicated to clinical application in humans, provided by Cobra Biomanufacturing: the pORT-RDD plasmid. This plasmid is also characterized by a strong constitutive promoter (CMV-intronA), a human secretion signal (human urokinase uPA signal), a reduced number of immunostimulatory CpG sequences (150 in pORT-RDD), a small size and high copy number. The pORT-RDD vector was unexpectedly found to achieve high level of expression of RDD transgene in vitro, associated with a strong antiproliferative activity (Figures 2 and 3).

Such plasmid is further described in international application W02008/107462 incorporated herein by reference.

### FIGURE

Figure 1: Structure of ADAM-15 and experimental plasmids.
   a) Domain structure of ADAM-15 (metargidin) and sequence alignment of RDD (SEQ ID NO:2) with the disintegrin domains of human (SEQ ID NO:2) and murine ADAM-15 (SEQ ID NO:3). * indicates amino acids shared in all three sequences.
   b) Map with restriction sites of the pVAX1 vector with inserted RDD or RDD-RFP, respectively. The pVAX vector contains 2999 bp (CMV promoter: bases 137-724; T7 promoter/priming site: 664-683; multiple cloning site: 696-811; BGH reverse priming site: 823-840; BGH polyadenylation signal: 829-1053; kanamycine resistance gene: 1226-2020; pUC origin: 2320-2993).
Figure 2: is the map of pORHaCMV plasmid.
Figure 3: is the map of pORT-RDD plasmid.

### DETAILED DESCRIPTION OF THE INVENTION

### Definitions

"Metargidin" denotes the human metalloprotease-RGD-disintegrin protein which also called MDC-15 or ADAM-15. Cloning of metargidin was described by Kratzschmar et al. (J Biol Chem 1996; 271:4593-6).

"RDD" denotes the disintegrin domain of human metargidin. The cDNA sequence of RDD is shown in the annexed sequence listing as SEQ ID NO:1.

"AMEP" or "Antiangiogenic Metargidin Peptide" denotes recombinant RDD peptide.

The term "derivatives" means a variant of the nucleic acid sequence, e.g. cDNA sequence of RDD in which one or more nucleotides or nucleic acids are substituted, added or deleted from the abovementioned sequence, as long as it retains a biological activity of wild-type RDD, e.g. inhibition of endothelial cell proliferation and/or inhibition of angiogenesis in vitro and/or in vivo. It is preferred that such a variant has a sequence with an identity of at least 80%, preferably at least 85 %, at least 90%, more preferably at least 95%, or at least 98 % identity with the abovementioned cDNA sequence SEQ ID NO: 1.

Inhibition of endothelial cell proliferation may be assessed, for instance, by culturing human umbilical vein endothelial cells (HUVEC) in the presence of increasing amounts of the fragment of derivative of RDD or in the absence thereof, for instance for 96 h, and by performing a cell proliferation assay to quantify inhibition of cell proliferation. Cell proliferation may be assessed by any suitable method known to the one skilled in the art, such as by means of MTT assay (e.g. cell proliferation Kit I, Roche Diagnostics, Germany). Inhibition of angiogenesis may be assessed, for instance, by assaying formation of tubule-like structures by endothelial cells cultured on a supportive matrix, such as Matrigel.

The term "peptide" is indifferently meant for a dipeptide, an oligopeptide, or a polypeptide, i.e. a polymer in which the monomers are amino acid residues joined together through amide bonds, whatever its length. The term derivative denotes a peptide which present a different amino acids sequence than SEQ ID n°1 but still present its functional properties.

"Plasmids" are extra-chromosomal double-stranded DNA (dsDNA) 5 molecules which are typically capable of autonomous replication within their hosts, for instance bacteria. As used herein, the terms "vector" and "plasmid" indifferently denote the vehicle by which RDD cDNA sequence can be introduced into a host cell, so as to transform the host and promote expression (e.g. transcription and translation) of the RDD sequence.

A "pORT plasmid" denotes a plasmid comprising an operator liable to binding by a repressor expressed in trans. Said operator may be for instance lac operator, A operator, trp operator, gal operator, ara operator, Arg operator and Tet operator. pORT plasmids have been described in Williams et al., Nucleic Acids Res. 1998 May 1;26(9):2120-4 ; Cranenburgh et al., Nucleic Acids Res. 2001 Mar 15 1;29(5):E26 ; Cranenburgh et al., J Mol Microbiol Biotechnol. 2004;7(4):197-203 and in the international patent application WO 9709435.

### EXAMPLE 1 : MATERIAL & METHODS

### 1. Plasmid preparation

The plasmids pVAX1 (Invitrogen, Taastrup, Denmark), pVAX-RDD, pVAX-RFP, pVAX-RDD-RFP (Fig.1a) were used. pVAX-RFP contained a pRFP-Monomer-N1 derived from the tetrameric Discosoma RFP (Clontech, Palo Alto, CA). RFP and/or RDD were cloned into the pGA4 (ampR) site of pVAX1. The plasmids were amplified in one shot® TOP10F' E. coli competent cells (Invitrogen) and prepared using the EndoFree Plasmid Mega or GigaKit (Qiagen, Hilden, Germany) or the NucleoBond® XtraMaxiKit (Macherey-Nagel, Bethlehem, PA). Plasmid concentration and quality were controlled by spectrophotometry and gel electrophoresis, and confirmed by sequencing.

### 2. Cells and functional in vitro assays

HUVEC (Cambrex, Vervier, Belgium) or CPAE endothelial cells were cultured in Endothelial Cell Growth Medium (PromoCell, Heidelberg, Germany). Human fibroblasts were cultured in MEM and murine LL/2 cells (LGC Standards, Middlesex, UK) in RPMI1640. The Nucleofector NHDF-Kit (Amaxa, Cologne, Germany) was used for fibroblast transfections and HiPerFect (Qiagen) for LL/2 cells. Transfection efficiencies were determined by FACS. HUVEC migratory activity was assessed for up to 29h in a standardized scratch-wound assay in 6-well plates using conditioned medium from different transfectants.

### 3. Mouse models

Experiments using human skin were approved by the Danish National Committee on Biomedical Research Ethics and informed consent was obtained from the patients. Animal experiments were approved by the governmental authorities (Regierung von Unterfranken or Danish Experimental Animal Inspectorate). Mice were housed under SPF conditions.

Mice with epidermal expression of latent human TGFβ1 (K5.TGFβ1) were genotyped by PCR. All experiments were performed with offspring from wild-type females and transgenic males, wild-type littermates served as controls. Transgenic and wild-type mice were used at 4-8 weeks of age. All mice were monitored in a blinded and standardized manner. For xenotransplantation, psoriasis patients without systemic treatment or phototherapy for two months and without topical treatment for at least 14 days were biopsied using a keratome (0.2mm; 4 biopsies of involved psoriatic skin from 4 patients (for the active psoriasis model) and 4 biopsies of non-involved skin from 4 patients (for the inductive model)). Mice were anaesthetized by intraperitoneal injection with 100mg/kg ketamine (Parke-Davis, Barcelona, Spain) and 20mg/kg xylazine (Bayer, Leverkusen, Germany) in 0.5ml PBS. Skin pieces were transplanted onto the back of Prkdcscid mice (Taconic, Ry, Denmark), and the wounds were closed using histoacryl® (TissueSeal, MI, USA). To induce psoriasis in non-lesional xenografts, 107 autologous PBMCs activated by 20U/ml IL-2 and 1µg/ml SEB (Sigma-Aldrich, Broendby, Denmark) were injected intraperitoneally after 14 days.

### 4. Intramuscular DNA electrotransfer

Gene therapy was performed in 6-12 weeks-old K5.TGFβ1 transgenic mice, in Prkdcscid mice on day 12 after transplantation of non-lesional skin (2d before PBMC injection), or on day 14 after transplantation of involved psoriatic skin. The mice were anaesthetized and 50µg plasmid DNA in 30µl PBS was injected into each tibialis cranialis muscle. Plate electrodes (4mm) and a specially modified Cliniporator™ (IGEA, Carpi, Italy) were used to apply one high-voltage (700V/cm, 100µs) and eight low-voltage pulses (100V/cm, 50ms).

### 5. Histology and immunohistochemistry

Paraffin-embedded tissues were stained by haematoxylin and eosin according to standard protocols. For immunohistochemistry of murine tissues, cryostat-cut sections (4µm) were stained as described. For immunohistochemistry of human tissue, 4µm paraffin-embedded sections were stained. Antibodies against the following antigens were used: mCD31 (clone MEC13.3), mCD51 (RMV-7), mCD49e (HM□5; all from BD Biosciences, Heidelberg, Germany), RFP (AB34771; Abcam, Cambridge, UK), Ki67 (MIB-1; Dako, Glostrup, Denmark), hCD3 (UCHT1; Dako), hCD31 (JC70A; Dako), hαSMA (1A4; Dako). Furthermore, Bandeirea simplicifolia-I-agglutinin (BS-1-lectin), staining vascular endothelium in many species except human, was used. In negative controls, primary antibodies were replaced with appropriate control antibodies. As a positive control, a colon adenocarcinoma was used for the Ki67 stainings, normal human and murine skin samples for the BS-1-stainings and a multiblock of liver, kidney and tonsil for the other stainings. Samples were analyzed in a blinded fashion by light and fluorescence stereomicroscopy (Leica, Wetzlar, Germany or Zeiss, Göttingen, Germany). The epidermal thickness was measured at 10 randomly chosen sites in each sample (Leica, LAS EZ1.6.0, Heerbrugg, Switzerland), and averages of the measurements were calculated. Keratinocyte proliferation was determined by counting Ki67+ cells per mm epidermis.

### 6. Flow cytometry

Antigen expression was determined using antibodies against the following antigens: CD49e (SAM1; Beckmann-Coulter, Krefeld, Germany), CD51 (13C2; Chemikon, Hofheim, Germany) and CD51/CD61 (LM609; Chemikon). Cells were analyzed using a FACSCanto II and the DIVA software (BD Biosciences).

### 7. Reverse transcriptase-PCR

Expression of RDD-encoding mRNA was analyzed by semi-quantitative RT-PCR. Total RNA was extracted using the E.Z.N.A. RNA-Kit (Omega, Norcross, GA, USA). Thereafter, cDNA was generated using the First Strand cDNA-Synthesis Kit (Fermentas, Leon-Rot, Germany) and an oligo(dT) primer. For amplification, Sawady-Taq (PeqLab, Erlangen, Germany) and sequence-specific primers (forward 5'-GCGCCTTGGTGGTGAAAAAC-3'; reverse 5'- AGTCATCCAGGAAGCCA-3') were used. PCR was performed in a Mastercycler® (Eppendorf, Hamburg, Germany) as follows: 5 min at 94°C, followed by 30 cycles of 30 seconds at 94°C, 30 seconds at 60°C and 5 min at 72°.

### 8. In vivo blood flow

The Vevo770 Imaging System equipped with a RMV708 Scanhead and a 3D motor (VisualSonics, Toronto, Canada) was used for high-resolution Doppler ultrasound in 4 areas in each mouse in a standardized fashion.

### 9. Statistics

Statistical differences were determined by X² - test or Student's t-test, both two-tailed. P-values of <0.05 (CI 95%) were considered significant. Data are displayed as mean ±SD.

### EXAMPLE 2 : A RECOMBINANT DISINTEGRIN DOMAIN (RDD) INHIBITS ENDOTHELIAL CELLS IN VITRO

ADAM-15 is evolutionary highly conserved, the disintegrin domains of murine and human ADAM-15 share amino acid sequence homologies of 77.6%. A recombinant polypeptide (RDD) homologous to murine, human and bovine ADAM-15 was generated (Fig.1). It interferes with α₅β₁ and αᵥβ₃ integrins of either species. The inhibitory capacity of the recombinant RDD molecule was demonstrated on cultured endothelial cells. When monolayers were "wounded" by standardized scratching and closure of the cell-free areas was monitored, recombinant RDD significantly inhibited the migration of endothelial cells in a dose-dependent fashion. In four independent experiments (16-21 individual cultures for each condition), endothelial cell migration in the presence of 15µg/ml RDD protein was reduced to 54.5% (±3.44; *p*<0.0001) as compared to untreated control culture.

To demonstrate that RDD secreted by transfected cells impaired endothelial cell functions, normal human fibroblasts were transfected by electroporation with different constructs. The RDD-encoding sequence and several control sequences were cloned into the *pVAX1* expression vector (Fig.1). The respective plasmids, *pVAX-mock, pVAX-RFP* (red fluorescent protein), *pVAX-RDD* and *pVAX-RDD-RFP,* were obtained from E. coli bacteria following transformation. Each plasmid construct was sequenced and no sequence alterations were observed (data not shown). Expression of the RFP-coupled fusion protein of RDD *(pVAX-RDD-RFP)* as well as the control RFP *(pVAX-RFP)* could be readily detected within transfected murine cells by fluorescence microscopy. Morphological changes of the transfectants were not detected. HUVEC monolayers, which strongly expressed the RDD target proteins, integrins αᵥβ₃ and α₅β₁, were incubated with supernatant from different transfected fibroblast populations, and closure of the cell-free lane (artificial "wound" generated by standardized scratching) was monitored microscopically for up to 29h. Cultures incubated with supernatant from RDD-expressing transfectants, but not controls, showed markedly delayed migration of endothelial cells into the gap (p<0.0001 at all measured timepoints, i.e., 6h, 12h and 24h), indicating functional activity of RDD secreted by transfectants. RDD target proteins were highly expressed in HUVEC cells.

### EXAMPLE 3 : EXPRESSION OF RDD IN VIVO

The next series of experiments was designed to achieve *in vivo* expression of the antiangiogenic peptide by non-viral gene therapy. Towards this end, the plasmids were transfected into the calf muscles *(m. tibialis cranialis)* of mice by DNA electrotransfer using an adjusted protocol with an initial high-voltage (700V/cm, 100µs) and eight subsequent low voltage (100V/cm, 50ms) pulses. Fluorescence microscopy demonstrated clear red signals within the calf muscles of mice transfected with the *pVAX-RDD-RFP* fusion protein or *pVAX-RFP.* Time course experiments revealed that expression of the transgenes was detectable as early as 24h after electrotransfer, reached a peak after three days, and tapered off within 2-3 weeks. Inflammatory reactions at the transfection sites or other unwanted side effects were not observed. Of note, when organs distant from the transfection site were analyzed in mice transfected with the *pVAX-RDD-RFP* control construct, clear fluorescence signals were detected within the cutaneous connective tissue, but not the epidermis. In contrast, control animals transfected with RFP alone or mock-transfected animals did not show dermal fluorescence, suggesting that RDD-containing peptides became deposited within the skin. The results obtained by fluorescence microscopy were confirmed by immunohistochemistry, again showing deposition of the RDD-containing products, but not the controls, in the vicinity of some blood vessels. Deposition of transgenes in other organs including muscle, liver and kidneys, was weak and morphological alterations in other organs were not detected.

### EXAMPLE 4 : IN VIVO RDD GENE THERAPY STOPS PSORIASIFORM CHANGES IN K5.TGFβ1 TRANSGENIC MICE

Having established that non-viral gene transfer could be successfully applied in *vivo,* the impact of RDD expression on cutaneous vascularization and the course of the psoriasis-like disorder in K5. TGFβ1-transgenic mice was assessed. In a series of preparatory experiments, it was established that K5.TGFβ1 transgenic mice showed significantly increased size and density of cutaneous blood vessels. As an independent second method, blood flow within the upper plexus of the skin was assessed at several time points for up to twelve days by high-resolution ultrasound. In order to achieve a reliable and reproducible functional quantification of the cutaneous blood flow (which shows considerable site-dependent variability), four areas distant from the transfection sites (two on the abdomen and one on each thigh) were analyzed in a standardized manner, and the mean blood flow from all four areas was calculated for each mouse. Again, this standardized functional approach demonstrated significantly increased vascularization and blood flow in K5.TGFβ1-transgenic mice compared to wild-type mice (14.3% of the total dermal volume (±4.1) in wild-type and 26.9% (±5.4) in K5.TGFβ1-transgenic mice; p<0.001, Fig.3b).

To determine the optimal concentration of RDD plasmid for transfections, we injected anaesthetized K5. TGFβ1-transgenic mice intramuscularly with 3 different doses of the *pVAX-RDD* plasmid (12.5, 25 or 50µg per hind leg, the latter dose being the highest that was tolerable without injection-related side effects) and performed electrotransfer. Given that 50µg of *pVAX-RDD* plasmid yielded the strongest diminution of the cutaneous blood flow, all experiments were performed using 50µg of plasmid in each calf muscle.

When K5. TGFβ1-transgenic mice (n=8 mice per group) were subjected to electroporation only (PBS control), *pVAX-* (mock transfection control) or *pVAX-RDD-*electroporation and monitored for 12 days, some remarkable effects of RDD on the psoriasiform phenotype became apparent: While the disease severity score showed the expected worsening in both control groups (scores 1.15 (±0.25) and 1.20 (±0.17), respectively, by the end of the observation period), disease progression was completely stopped in the group treated by RDD gene therapy (score 0.56 (±0.25); p=0.0034 compared to the *pVAX-mock* control group). End-point analysis by immunohistochemistry and subsequent morphometric analysis confirmed the *in vivo* observations: RDD gene therapy resulted in significantly reduced cutaneous vascularization (overall reduction of CD31-positive blood vessels by 25% as compared to either of the controls; p=0.0053). Likewise, gene therapy with RDD led to significantly reduced epidermal thickness (reduction by 32%, p=0.0041).

### EXAMPLE 5 : ANTI-ANGIOGENIC RDD GENE THERAPY PREVENTS PSORIASIS IN AN INDUCIBLE XENOTRANSPLANTATION MODEL

Having shown that RDD gene therapy was efficacious in K5. TGFβ1 transgenic mice, we next assessed whether this antiangiogenic gene therapy would affect the pathogenesis of human psoriasis in the psoriasis/SCID mouse xenograft model, in which human skin was transplanted onto *Prkdc^{scid}* mic, and which allows the study of psoriasis development in connection with angiogenesis. After several preliminary experiments in which it was established that *in vivo* RDD expression can be used in the xenotransplantation models, non-involved skin from psoriasis patients (four patients, each biopsied piece of skin yielding >6 transplants) was transplanted, and psoriasis was induced by injection of SEB and IL-2-stimulated autologous PBMC (37, 43). The DNA electrotransfer using 50µg of *pVAX-RDD* or *pVAX-RFP* (into both *tibialis cranialis* muscles) was performed two days prior to the induction of psoriasis. Disease development was monitored for 14 days.

Psoriasis induction in the transplants was accompanied by profoundly increased vascularization both macroscopically and histopathologically. Given that the grafts may harbor blood vessels of both human and murine origin, we evaluated the effect of RDD using several staining methods. Human-derived vessels were detected using antibodies directed against human CD31 (PECAM-1) and α-SMA. Murine endothelial cells were stained by anti-mouse BS-1-lectin. Of note, gene therapy with RDD resulted in marked reduction of cutaneous blood vessels compared to control-treated mice.

When other features of psoriasis were assessed morphometrically, highly significant effects were observed regarding epidermal thickness and numbers of proliferating cells detected by reactivity with the Ki67 mAb. The epidermal thickness (measured in 8-10 randomly chosen sites in the middle of each graft in a blinded fashion) was 162.5µm (SD=9.7) in the control *pVAX-RFP-treated* mice (n=9) and 89.7µm (SD=9.8) in the *pVAX-RDD* treated mice (n=15; reduction by 45%; p=0.000034). In addition, the numbers of proliferating epidermal cells showed highly significant differences between controls and RDD-treated mice (121.5 cells/mm (SD=15.9) in *pVAX-RFP-treated* mice and 42.9 cells/mm (SD=6.0) in *pVAX-RDD-*treated control mice; reduction by 65%; p=0.0012), indicating that the primary effect of RDD gene therapy on cutaneous vascularization indeed alleviated typical epidermal features of psoriasis. Cutaneous T-cells were not significantly different in RDD-treated versus control-treated mice.

### EXAMPLE 6 : THERAPEUTIC EFFICACY OF RDD GENE THERAPY ON FULLY DEVELOPED PSORIATIC PHENOTYPE

The impact of halting angiogenesis by RDD gene therapy on full-fledged human psoriatic skin (four patients, each biopsy specimen yielding >8 transplants) was assessed. The treatment was administered 14 days after transplantation as intramuscular injection of 50µg *pVAX-RDD* or the control *pVAX-RFP* in both m. *tibialis cranialis,* and subsequent electroporation. The conspicuously prominent vascularization visible on the undersides of the transplanted skin flaps in the control mice was markedly reduced in mice treated by RDD gene therapy. This macroscopic feature was paralleled by markedly reduced vascularization on the microscopic level, indicating that *in vivo* expression of the RDD fragment caused diminution of number and size of the hypertrophic cutaneous microvessels.

Of note, RDD transfection also led to significantly reduced epidermal thickness and numbers of proliferating cells compared to the controls. The epidermal thickness was 166.85µm (SD=12.1) in *pVAX-RFP-treated* mice (n=14 animals) and 88.29µm (SD=7.7) in *pVAX-RDD*-treated mice (n=13 animals); *p*=0.000021). Likewise, the numbers of proliferating epidermal cells were significantly reduced in RDD-treated mice (312.0 cells/mm (SD=48.3) in *pVAX-RFP*-treated and 42.5 cells/mm (SD=14.2) in *pVAX-RDD*-treated mice; p=0.0019). Thus, antiangiogenic gene therapy was highly efficacious both in the prevention and in the treatment of psoriasis lesions *in vivo.*

## Claims

1. Disintegrin domain of an adamalysin for its use to treat and/or prevent psoriasis or other inflammatory diseases.

2. Disintegrin domain of an adamalysin according to claim 1 wherein the disintegrin domain is a nucleic acid molecule comprising or constituting by a polynucleotide sequence coding all or part of the disintegrin domain of an adamalysin or a derivative thereof

3. Disintegrin domain of an adamalysin according to claim 1 wherein the disintegrin domain is a polypeptide sequence comprising or constituted by all or part of the disintegrin domain of an adamalysin or a derivative thereof

4. Disintegrin domain of an adamalysin according to anyone of claim 1 to 3 wherein the adamalysin is metargidin.

5. Disintegrin domain of an adamalysin according to anyone of claim 1 to 4 wherein it comprises in part or entirely the disintegrin domain of metargidin having the nucleotidic sequence represented in SEQ ID NO: 1 or a derivative thereof.

6. Disintegrin domain of an adamalysin according to anyone of claim 1 to 5 wherein it comprises in part or entirely the disintegrin domain of metargidin having the amino acid sequence represented in SEQ ID NO: 2 or a derivative thereof.

7. Disintegrin domain of an adamalysin according to anyone of claim 1 to 6 for wherein it is administered through electrotransfer.

8. Disintegrin domain of an adamalysin according to claim 7 wherein the electrotransfer method comprises:
- at least one pulse of a High Voltage (HV) field strength of between 200 and 2000 volts/cm for a duration of 10 to 1000 µs and;
- at least one second pulse of Low Voltage (LV) field strength of between 50 and 200 volts/cm and of duration of between 10 and 2000 ms.

9. Disintegrin domain of an adamalysin according to anyone of claim 7 or 8 wherein the at least one second pulse is 1-15 pulses, preferably 2-10 pulses, more preferably 6-10 pulses, typically 8 pulses.

10. Disintegrin domain of an adamalysin according to anyone of claim 7 to 9 wherein the Low Voltage (LV) field strength is of 50-200, preferably 80-120, typically about 100 volts/cm.

11. Disintegrin domain of an adamalysin according to anyone of claim 7 to 10 wherein the the Low Voltage pulse(s) is between 10 and 2000 ms, preferably 10 and 500 ms, more preferably 10 and 50 ms, typically about 50 ms.

12. Disintegrin domain of an adamalysin according to anyone of claim 7 to 11 wherein the electrotransfer method comprises:
- one pulse of High Voltage (HV) field strength of about 700 volts/cm for a duration of about 100 µs and
- 8 pulses of Low Voltage (LV) field strength of about 100 volts/cm and of duration of about between 50 ms.

13. Disintegrin domain of an adamalysin according to anyone of claim 1 to 12 wherein the treated and/ or prevented psoriasis is psoriatic arthritis.


SEQUENCE LISTING

<110>  BIOALLIANCE PHARMA

<120>  Use of disintegrin domain of an adamalysin for the treatment of
       psoriasis

<130>  BET 10L0127

<160>  3

<170>  PatentIn version 3.4

<210>  1
<211>  276
<212>  DNA
<213>  Homo sapiens


<220>
<221>  misc_feature
<222>  (1)..(276)
<223>  RDD

<400>  1
atggctgctt tctgcggaaa tatgtttgtg gagccgggcg agcagtgtga ctgtggcttc      60

ctggatgact gcgtcgatcc ctgctgtgat tctttgacct gccagctgag gccaggtgca     120

cagtgtgcat ctgacggacc ctgttgtcaa aattgccagc tgcgcccgtc tggctggcag     180

tgtcgtccta ccagagggga ttgtgacttg cctgaattct gcccaggaga cagctcccag     240

tgtccccctg atgtcagcct aggggatggc gagtaa                               276


<210>  2
<211>  91
<212>  PRT
<213>  Homo sapiens

<400>  2

Met Ala Ala Phe Cys Gly Asn Met Phe Val Glu Pro Gly Glu Gln Cys
1               5                   10                  15


Asp Cys Gly Phe Leu Asp Asp Cys Val Asp Pro Cys Cys Asp Ser Leu
            20                  25                  30


Thr Cys Gln Leu Arg Pro Gly Ala Gln Cys Ala Ser Asp Gly Pro Cys
        35                  40                  45


Cys Gln Asn Cys Gln Leu Arg Pro Ser Gly Trp Gln Cys Arg Pro Thr
    50                  55                  60


Arg Gly Asp Cys Asp Leu Pro Glu Phe Cys Pro Gly Asp Ser Ser Gln
65                  70                  75                  80


Cys Pro Pro Asp Val Ser Leu Gly Asp Gly Glu
                85                  90


<210>  3

<211>  91
<212>  PRT
<213>  Mus musculus

<400>  3

Met Ser Ser Leu Cys Gly Asn Met Phe Val Asp Pro Gly Glu Gln Cys
1               5                   10                  15

Asp Cys Gly Phe Pro Asp Glu Cys Thr Asp Pro Cys Cys Asp His Phe
            20                  25                  30

Thr Cys Gln Leu Arg Pro Gly Ala Gln Cys Ala Ser Asp Gly Pro Cys
        35                  40                  45

Cys Gln Asn Cys Lys Leu His Pro Ala Gly Trp Leu Cys Arg Pro Pro
    50                  55                  60

Thr Asp Asp Cys Asp Leu Pro Glu Phe Cys Pro Gly Asp Ser Ser Gln
65                  70                  75                  80

Cys Pro Ser Asp Ile Arg Leu Gly Asp Gly Glu
                85                  90